(19) **Europäisches Patentamt**
**European Patent Office**
**Office européen des brevets**

(11) **EP 4 613 324 A1**

(12) **EUROPEAN PATENT APPLICATION**
published in accordance with Art. 153(4) EPC

(43) Date of publication:
**10.09.2025 Bulletin 2025/37**

(51) International Patent Classification (IPC):
***A61N 5/10*** (2006.01)

(21) Application number: **23887942.3**

(22) Date of filing: **06.11.2023**

(52) Cooperative Patent Classification (CPC):
**A61N 5/10; G06T 17/00; G06V 10/25; G16H 30/00;**
**G16H 50/50; G16H 70/20; G16H 70/40**

(86) International application number:
**PCT/CN2023/129862**

(87) International publication number:
**WO 2024/099252 (16.05.2024 Gazette 2024/20)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **07.11.2022 CN 202211386001**
**05.09.2023 CN 202311141034**

(71) Applicant: **Neuboron Therapy System Ltd.**
**Xiamen, Fujian 361026 (CN)**

(72) Inventors:
• **LIU, Yuanhao**
**Nanjing, Jiangsu 211112 (CN)**
• **TENG, Yi-chiao**
**Nanjing, Jiangsu 211112 (CN)**
• **CHEN, Jiang**
**Nanjing, Jiangsu 211112 (CN)**

(74) Representative: **Gunzelmann, Rainer**
**Wuesthoff & Wuesthoff**
**Patentanwälte und Rechtsanwalt PartG mbB**
**Schweigerstraße 2**
**81541 München (DE)**

(54) **BORON NEUTRON CAPTURE TREATMENT SYSTEM AND TREATMENT PLAN GENERATION METHOD THEREFOR**

(57) Provided are a boron neutron capture treatment system (100) and a working method therefor. The boron neutron capture treatment system (100) comprises: a neutron beam irradiation device (10) for generating a neutron beam and irradiating an irradiated body; a treatment planning module (30) for assigning a boron concentration value to each voxel unit in a three-dimensional voxel prosthesis tissue model and generating a treatment plan; and a control module (40) for controlling the neutron beam irradiation device (10) to execute irradiation according to the treatment plan. The treatment planning module (30) assigns corresponding boron concentration data to each voxel unit, and then performs dose simulation in combination with medical image data to formulate the treatment plan, such that the distribution of boron atoms in a region of interest better conforms to the actual situation, the precision of model establishment and dose calculation can be improved, and the accuracy of the treatment plan is ensured, thereby ensuring the treatment effect.

| Neutron beam irradiation device 10 | | Treatment planning module 30 | Control module 40 |
|---|---|---|---|
| Neutron generation device 11 | Treatment table 12 | | |

**FIG. 3**

**Description**

## TECHNICAL FIELD

The present disclosure relates to the technical field of radiotherapy, and in particular to a boron neutron capture therapy (BNCT) system and a treatment plan generation method thereof.

## BACKGROUND

With the development of atomics, the radiotherapy such as the cobalt-60, the linear accelerator, and the electron beam has become one of major means to treat cancers. However, the conventional photon or electron therapy is restricted by physical conditions of radioactive rays. Specifically, while tumor cells are killed, a large number of normal tissues on a beam path are damaged. Due to different sensitivities of the tumor cells for the radioactive rays, the conventional radiotherapy is often undesirable to treat radioresistant malignant tumors (such as glioblastoma multiforme and melanoma).

In order to reduce radiation damages to the normal tissues surrounding the tumor, the target therapy in chemotherapy has been employed in the radiotherapy. For the highly radioresistant tumor cells, the radiotherapy with high relative biological effectiveness (RBE), including the proton therapy, the heavy particle therapy, and the neutron capture therapy, has also been developed actively. With specific aggregation of boron-containing drugs in tumor cells, and in cooperation with accurate neutron beam control, BNCT in neutron capture therapy serves as a better alternative to treat the cancers.

According to the BNCT, with a large capture cross section of the boron-containing drug for thermal neutrons, and through the $^{10}B(n,\alpha)^7Li$ neutron capture reaction and the nuclear fission reaction, $^4He$ and $^7Li$ heavy charged particles are generated. The two heavy charged particles have an average energy of about 2.33 MeV, and have characteristics of the high linear energy transfer (LET), and the short range. The alpha particle has the LET of 150 keV/$\mu$m, and the range of 8 $\mu$m. The $^7Li$ heavy charged particle has the LET of 175 keV/$\mu$m, and the range of 5 $\mu$m. The total range of the two particles is approximately equivalent to a cell size, such that the radiation damage to an organism is limited to a cell level. When the boron-containing drug is aggregated to the tumor cells selectively, and an appropriate neutron source is provided, a purpose of locally killing the tumor cells on premise of no serious damage to normal tissues can be achieved.

In the BNCT, since the neutron beam for irradiating radioactive rays onto the irradiated body for treatment has the strong radioactive rays, an irradiation dose on the irradiated body is to be controlled accurately, so as to achieve the better treatment effect, and reduce the radiation damage of the radioactive rays on the irradiated body as much as possible. Hence, the accurate formulation of the treatment plan is crucial. The three-dimensional (3D) model can simulate an absorbed dose of a human body under certain radiation conditions to facilitate formulation of the treatment plan. Usually, medical image data is processed repeatedly with a computer technology, so as to establish an accurate lattice model required by the Monte Carlo software, and perform simulation and computation with the Monte Carlo software. In the field of the neutron capture therapy, when the lattice model required by the Monte Carlo software is established according to the medical image data, and the dose is computed and evaluated, basic information of the organism reflected by each lattice, such as a tissue type and a boron concentration, is defined in the model. The reliable computed result depends on the accuracy and precision of the information. By testing a blood sample or a slice, a boron concentration in the tissue and a boron concentration in the tumor are estimated correspondingly, thereby giving the boron concentration to the corresponding region of the model. For the given boron concentration, neither the real distribution of the boron-containing drug in the organism nor the metabolism of the boron-containing drug over time is considered. This affects the reliability of the computed dose result to cause the low accuracy of the treatment plan and the undesired treatment effect.

## SUMMARY

In view of the above-mentioned technical problems, the present disclosure provides a BNCT system capable of ensuring a treatment effect and a treatment plan generation method thereof.

According to a first aspect, the present disclosure provides a BNCT system, including a neutron beam irradiation device configured to generate a neutron beam and irradiate an irradiated body; a treatment planning module configured to perform boron concentration assignment on each voxel in a 3D voxel prosthesis tissue model and generate a treatment plan; and a control module configured to control the neutron beam irradiation device according to the treatment plan for irradiation.

Further, the treatment planning module is configured to perform group division on a region of interest (ROI) in the 3D voxel prosthesis tissue model based on standardized uptake values (SUVs) and compute an average of SUVs in each group.

Further, the average of SUVs in each group is computed by:

$$\overline{SUV}_{ROI,i} = \frac{SUV_{ROI,upper} - SUV_{ROI,lower}}{I} \times (i - 0.5) + SUV_{ROI,lower} \quad (Eq.\ 2)$$

$$i = 1, 2, 3, 4, \dots, I$$

where, I is a number of groups, and is an even number in an embodiment of the present disclosure, $SUV_{ROI,upper}$ is an upper limit of SUVs in the ROI, and $SUV_{ROI,lower}$ is a lower limit of the SUVs in the ROI.

Further, the number I of groups is greater than or equal to 10 and less than or equal to 500.

Further, the number I of groups is greater than or equal to 10 and less than or equal to 100.

Further, the number I of groups is 40, 44, 50, 54 or 60.

Further, the treatment planning module is configured to adjust the number I of groups or the average of SUVs based on a normalization factor k; and the normalization factor k is computed by:

$$\int_V SUV_{ROI}(V)dV = k \sum_i N_i \overline{SUV}_{ROI,i}$$

$$(Eq.\ 3)$$

$$k = \frac{\int_V SUV_{ROI}(V)dV}{\sum_i N_i \overline{SUV}_{ROI,i}}$$

where, $SUV_{ROI}(V)$ is an SUV of a Vth voxel in the ROI, $\int_V SUV_{ROI}(V)dV$ is a total value of SUVs before the group division, $N_i$ is a count of an ith group, k is the normalization factor, and $\sum_i N_i \overline{SUV}_{ROI,i}$ is a total value of SUVs after the group division.

Further, the treatment planning module is configured to determine a boron concentration $N_{B10}$,group(V) of each voxel in the ROI by:

$$N_{B10,group}(V) = \xi \times k \times \overline{SUV}_{ROI,i}$$

$$i = \left\lceil \frac{I \times (SUV_{ROI}(V) - SUV_{ROI,lower})}{SUV_{ROI,upper} - SUV_{ROI,lower}} \right\rceil \quad (Eq.\ 4)$$

where, $\xi$ is a constant conversion factor, and is configured to convert each of SUVs into a corresponding number of $^{10}$B atoms, i represents a group index, and $SUV_{ROI}(V)$ is the SUV of the Vth voxel in the ROI.

According to a second aspect, the present disclosure provides a BNCT system, including a neutron irradiation device configured to generate a neutron beam and irradiate an irradiated body; a treatment planning module configured to determine at least a high-concentration drug absorption region and a low-concentration drug absorption region based on an ROI of the irradiated body, and perform drug concentration assignment and dose evaluation on the high-concentration drug absorption region and the low-concentration drug absorption region to generate a treatment plan; and a control module configured to control the neutron irradiation device to execute the treatment plan.

Further, the treatment planning module includes:

a model establishment module configured to establish a 3D voxel tissue model based on medical image data of the irradiated body;
a processing module configured to define the ROI based on the 3D voxel tissue model, determine at least the high-concentration drug absorption region and the low-concentration drug absorption region based on the ROI, perform group division on the high-concentration drug absorption region and the low-concentration drug absorption region, the high-concentration drug absorption region being divided into at least one group, the low-concentration drug absorption region being divided into at least two groups, and assign a drug concentration to each group;
a dose evaluation module configured to perform the dose evaluation based on the drug concentration and an irradiation parameter of the neutron beam; and
a treatment plan generation module configured to generate the treatment plan based on a result of the dose evaluation.

Further, the treatment planning module is further configured to define a tumor-to-blood ratio (TBR) as a ratio of a drug concentration in the ROI to a drug concentration in blood, and define a tumor-to-normal tissue ratio (TNR) as a ratio of the drug concentration in the ROI to a drug concentration in a normal tissue; and

the processing module is configured to determine a region with a TBR greater than or equal to a first specified value or a region with a TNR greater than or equal to a second specified value in the ROI as the high-concentration drug absorption region, and determine a region with a TBR less than the first specified value or a region with a TNR less than the second specified value in the ROI as the low-concentration drug absorption region.

Further, a drug is a boron-containing drug; and when a boron concentration in the normal tissue is lower than a boron concentration in the blood, specifically, a TNR is greater than a TBR in a same ROI, the first specified value is greater than or equal to 1.2, and the second specified value is greater than or equal to 1.5.

Further, the drug is a boronophenylalanine (BPA); and for the BPA, a boron concentration in the normal tissue is approximate to a boron concentration in blood, specifically, a TNR is the same as a TBR in a same ROI, the first specified value is 2.5, and the second specified value is 2.5.

Further, the drug is a radionuclide labeled $^{18}$F-BPA.

Further, the processing module is configured to divide the ROI into three groups; the high-concentration drug absorption region is divided into one group, called a first region; the low-concentration drug absorption region is divided into two groups, respectively called a second region and a third region; the second region is a region with a TBR greater than or equal to a third specified value or a TNR greater than or equal to a fourth specified value in the low-concentration drug absorption region; the third region is a region with a TBR less than the third specified value or a TNR less than the fourth specified value in the low-concentration drug absorption region; the third specified value is greater than 1.5 and less than or equal to 2.0; and the fourth specified value is the same as the third specified value.

Further, the third specified value is the same as the fourth specified value, and is 2.0.

Further, the drug is a radionuclide labeled drug; and the treatment planning system is further configured to convert the drug concentration into one of a marker radioactive activity acquired based on the medical image data, a marker radioactive intensity, a number of atoms decayed in unit time, an effective count of annihilation photons, and an SUV for quantitative or semi-quantitative analysis.

Further, the drug is a radionuclide labeled boron-containing drug; and the treatment planning system is further configured to convert the drug concentration into the SUV for quantitative analysis:

$$N_{B10}(V) = \xi \times \overline{SUV_{ROI}(V)} \qquad \text{(Eq. 6)}$$

where, $N_{B10}(V)$ represents a number of $^{10}$B atoms in a Vth region, $\xi$ is a constant conversion factor, and $\overline{SUV_{ROI}(V)}$ is an average of SUVs in the Vth region of the ROI.

According to a third aspect, the present disclosure provides a treatment plan generation method of a BNCT system, including the following steps: establishing a 3D voxel prosthesis tissue model having a tissue type and a tissue density; performing boron concentration assignment on each voxel in the 3D voxel prosthesis tissue model; acquiring a dose distribution; and optimizing an irradiation angle according to a computed result to generate a treatment plan.

Further, the establishing a 3D voxel prosthesis tissue model having a tissue type and a tissue density includes: reading medical image data; establishing a 3D medical image voxel model; defining a boundary of an ROI; and defining a tissue type (element composition) and a tissue density of each voxel.

Further, the performing boron concentration assignment on each voxel in the 3D voxel prosthesis tissue model includes: performing group division on the ROI based on SUVs; and determining a boron concentration of each voxel in the ROI.

Further, the performing group division on the ROI includes: converting $^{10}$B information of each voxel in the ROI of an image into one of the SUVs by:

$$SUV_{\text{body weight}}(\text{kg/ml}) = \frac{Acitivity\ Concentration\ in\ ROI(Bq/ml)}{\left(\dfrac{Injected\ Dose(Bq)}{body\ weight(kg)}\right)} \qquad \text{(Eq.1)}$$

where, ROI is defined in the image, Acitivity Concentration in ROI is an average radioactive activity of each unit volume in the ROI, *Injected Dose* is an injected radioactive activity, and *body weight* is a body weight of the irradiated body.

Further, the performing group division on the ROI specifically includes: adjusting a number I of groups or an average of SUVs through a normalization factor k.

Further, the normalization factor k is computed by:

$$\int_V SUV_{ROI}(V)dV = k \sum_i N_i \overline{SUV}_{ROI,i}$$

$$(Eq. 3)$$

$$k = \frac{\int_V SUV_{ROI}(V)dV}{\sum_i N_i \overline{SUV}_{ROI,i}}$$

where, $SUV_{ROI}(V)$ is an SUV of a Vth voxel in the ROI, $\int_V SUV_{ROI}(V)dV$ is a total value of SUVs before group division, $N_i$ is a count of an ith group, k is the normalization factor, $\Sigma_i N_i \overline{SUV}_{ROI,i}$ is a total value of SUVs after the group division, $SUV_{ROI,i}$ is an average of SUVs of the ith group after the group division, and $SUV_{ROI,i}$ is computed by:

$$\overline{SUV}_{ROI,i} = \frac{SUV_{ROI,upper} - SUV_{ROI,lower}}{I} \times (i - 0.5) + SUV_{ROI,lower} \qquad (Eq. 2)$$

$$i = 1, 2, 3, 4, \dots, I$$

where, I is the number of groups.

Further, the boron concentration $N_{B10}$,group(V) of each voxel in the ROI is computed by:

$$N_{B10,group}(V) = \xi \times k \times \overline{SUV}_{ROI,i}$$

$$i = \left\lceil \frac{I \times (SUV_{ROI}(V) - SUV_{ROI,lower})}{SUV_{ROI,upper} - SUV_{ROI,lower}} \right\rceil \qquad (Eq. 4)$$

where, $\xi$ is a constant conversion factor, and is configured to convert each of SUVs into a corresponding number of $^{10}B$ atoms, i represents a group index, and $SUV_{ROI}(V)$ is the SUV of the Vth voxel in the ROI.

According to a fourth aspect, the present disclosure provides a treatment plan generation method of a BNCT system, including: establishing a 3D voxel tissue model based on medical image data of an irradiated body; defining an ROI based on the 3D voxel tissue model; determining at least a high-concentration drug absorption region and a low-concentration drug absorption region based on the ROI, performing group division on the high-concentration drug absorption region and the low-concentration drug absorption region, the high-concentration drug absorption region being divided into at least one group, and the low-concentration drug absorption region being divided into at least two groups, and assigning a drug concentration to each group; performing dose evaluation based on the drug concentration and an irradiation parameter of a neutron beam to obtain a dose distribution; and optimizing an irradiation angle according to a computed result to generate a treatment plan.

Further, the determining at least a high-concentration drug absorption region and a low-concentration drug absorption region based on the ROI includes: defining a TBR as a ratio of a drug concentration in the ROI to a drug concentration in blood, and defining a TNR as a ratio of the drug concentration in the ROI to a drug concentration in a normal tissue; and determining a region with a TBR greater than or equal to a first specified value or a region with a TNR greater than or equal to a second specified value in the ROI as the high-concentration drug absorption region, and determining a region with a TBR less than the first specified value or a region with a TNR less than the second specified value in the ROI as the low-concentration drug absorption region.

Further, a drug is a boron-containing drug, the first specified value is greater than or equal to 1.2, and the second specified value is greater than or equal to 1.5.

Further, the drug is a BPA, and the first specified value is the same as the second specified value, and is 2.5.

Further, the drug is a radionuclide labeled $^{18}F$-BPA.

Further, the ROI is divided into three groups; the high-concentration drug absorption region is divided into one group, called a first region; the low-concentration drug absorption region is divided into two groups, respectively called a second region and a third region; the second region is a region with a TBR greater than or equal to a third specified value or a TNR greater than or equal to a fourth specified value in the low-concentration drug absorption region; the third region is a region with a TBR less than the third specified value or a TNR less than the fourth specified value in the low-concentration drug absorption region; the third specified value is the same as the fourth specified value; and the third specified value is greater than 1.5 and less than or equal to 2.0.

Further, the third specified value is the same as the fourth specified value, and is 2.0.

Further, the drug is a radionuclide labeled boron-containing drug; and the drug concentration is converted into the SUV for quantitative analysis:

$$N_{B10}(V) = \xi \times \overline{SUV_{ROI}(V)} \qquad \text{(Eq. 6)}$$

where, $N_{B10}(V)$ represents a number of $^{10}$B atoms in a Vth region, $\xi$ is a constant conversion factor, and $\overline{SUV_{ROI}(V)}$ is an average of SUVs in the Vth region of the ROI.

Further, the defining an ROI includes: acquiring first image data of the irradiated body; acquiring second image data of the irradiated body; performing registration and alignment on the first image data and the second image data to obtain fused image data; and dividing the ROI based on the fused image data.

Further, the first image data is computed tomography (CT) data or magnetic resonance imaging (MRI) data, and the second image data is radionuclide medical image data.

According to the BNCT system provided by the first aspect and the third aspect of the present disclosure, the treatment planning module is configured to assign a corresponding boron concentration to each voxel, and perform dose simulation in combination with medical image data to formulate a treatment plan, such that the distribution of boron atoms in the ROI better conforms to an actual condition. This can improve the accuracy in the model establishment and the dose computation, ensure the accuracy of the treatment plan, and guarantee the treatment effect.

According to the BNCT system and the dose evaluation method thereof provided by the second aspect and the fourth aspect of the present disclosure, the ROI is divided into at least a high-concentration drug absorption region and a low-concentration drug absorption region. This prevents the problem that excessive groups cause a large number of boron concentrations to increase computation time in Monte Carlo simulation, and prevents the problem that the computational reliability is insufficient for assignment of the single boron concentration. Meanwhile, this further makes the dose evaluation more accurate and credible, and further optimizes the treatment plan. The present disclosure not only can ensure the accuracy of the dose computation, and make the lesion achieve an enough prescribed dose, but also can significantly reduce the amount of computation in the Monte Carlo simulation, shorten the computation time, generate the treatment plan more quickly and accurately, and improve the operation efficiency of the treatment system.

**BRIEF DESCRIPTION OF THE DRAWINGS**

FIG. 1 is a schematic view of a boron neutron capture reaction;
FIG. 2 illustrates an equation of a nuclear reaction in $^{10}$B(n,$\alpha$) $^{7}$Li neutron capture;
FIG. 3 is a block diagram of a BNCT system according to an embodiment of the present disclosure;
FIG. 4 is a schematic structural view of a neutron irradiation device according to an embodiment of the present disclosure.
FIG. 5 is a flowchart of a treatment plan generation method according to an embodiment of the present disclosure;
FIG. 6 is a flowchart of a method for establishing a 3D voxel prosthesis tissue model according to an embodiment of the present disclosure;
FIG. 7 is a flowchart of a method for giving a boron concentration to each voxel in a 3D voxel prosthesis tissue model according to an embodiment of the present disclosure;
FIG. 8 is a flowchart of dose evaluation in BNCT according to another embodiment of the present disclosure;
FIG. 9 is a schematic view of a CTsim image and a lesion region according to another embodiment of the present disclosure;
FIG. 10 is a schematic view of a fused image and an ROI according to another embodiment of the present disclosure;
FIG. 11 is a schematic view of region division of an ROI according to another embodiment of the present disclosure;
FIG. 12 is a TBR-volume histogram of an ROI according to another embodiment of the present disclosure; and
FIG. 13 illustrates a comparison with a dose-volume histogram (DVH) in a control group according to another embodiment of the present disclosure.

In the figures:
100: BNCT system, 10: neutron beam irradiation device, 11: neutron generation device, 12: treatment table, 20: image acquisition module, 30: treatment planning module, 40: control module, 100: neutron irradiation device, 110: radiation generation device, 111: accelerator, 112: beam transmission device, 120: beam shaper, 126: collimator, 121: reflector, 122: moderator, 123: thermal neutron absorber, 124: radiation shield, and 125: beam channel.

**DETAILED DESCRIPTION OF THE EMBODIMENTS**

To make the objectives, technical solutions, and advantages of the present disclosure clearer, the present disclosure is further described in detail below with reference to the drawings and embodiments. It should be understood that the specific embodiments described herein are merely used to explain the present disclosure, rather than to limit the present disclosure.

Preferably, an embodiment of the present disclosure provide a neutron capture therapy system and a treatment plan generation method thereof. The neutron capture therapy, particularly the BNCT, is described briefly below.

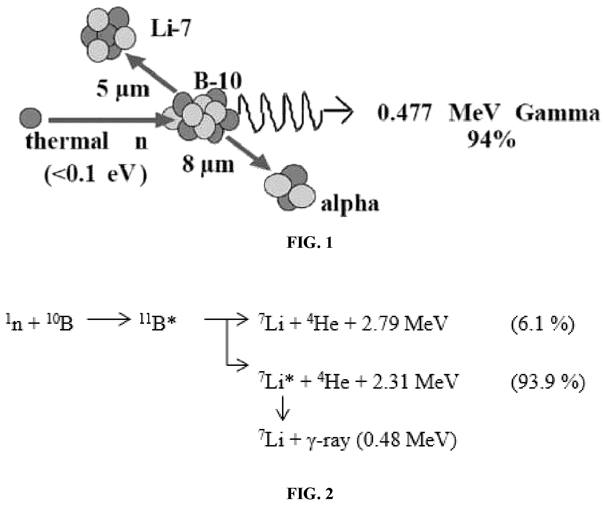

According to the BNCT, with a large capture cross section of the boron-containing drug for thermal neutrons, and through the $^{10}B(n,\alpha)^{7}$Li neutron capture reaction and the nuclear fission reaction, $^{4}$He and $^{7}$Li heavy charged particles are generated. FIG. 1 and FIG. 2 respectively illustrate a schematic view of a boron neutron capture reaction and an equation of a nuclear reaction in $^{10}B(n,\alpha)$ $^{7}$Li neutron capture. The two heavy charged particles have an average energy of about 2.33 MeV, and have characteristics of the high LET, and the short range. The alpha particle has the LET of 150 keV/$\mu$m, and the range of 8 $\mu$m. The $^{7}$Li heavy charged particle has the LET of 175 keV/$\mu$m, and the range of 5 $\mu$m. The total range of the two particles is approximately equivalent to a cell size, such that the radiation damage to an organism is limited to a cell level. When the boron-containing drug is aggregated to the tumor cells selectively, and an appropriate neutron source is provided, a purpose of locally killing the tumor cells on premise of no serious damage to normal tissues can be achieved.

The Monte Carlo method can accurately simulate collision trajectories and energy distributions of nuclear particles in a 3D space in the radiation object. In the BNCT, in order to simulate an absorbed dose of a human body under certain radiation conditions to facilitate formulation of the treatment plan, a medical image is processed repeatedly with a computer technology, so as to establish an accurate lattice model required by the Monte Carlo software, and perform simulation and computation with the Monte Carlo software. The medical image data may be MRI data, CT data, a positron emission tomography (PET) data, PET-CT data or X-ray imaging data. As is known to those skilled in the art, other medical image data may also be used in the radiotherapy system and the treatment plan generation method thereof in the present disclosure, provided that the medical image data can be converted into a 3D voxel prosthesis tissue model.

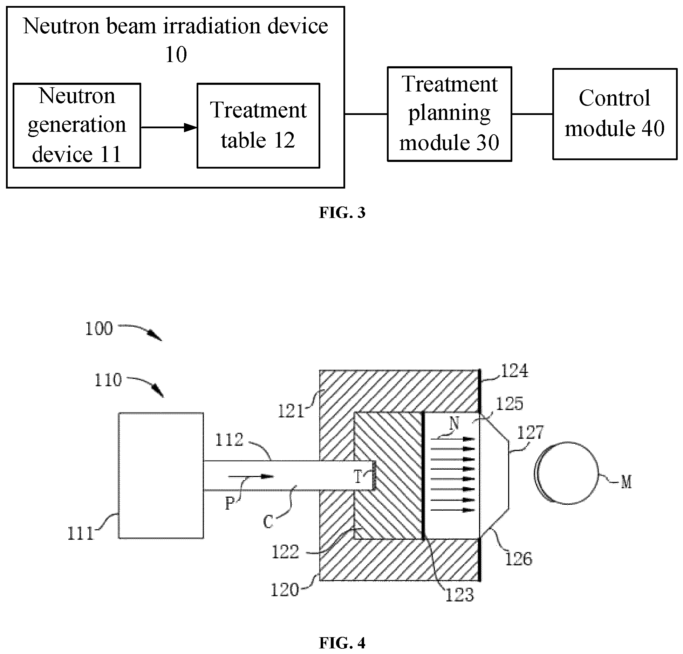

Referring to FIG. 3, an embodiment of the present disclosure provides a BNCT system 100, including a neutron beam irradiation device 10, an image acquisition module 20, a treatment planning module 30, and a control module 40. The neutron beam irradiation device 10 includes a neutron generation device 11 and a treatment table 12. The neutron generation device 11 is configured to generate a neutron beam N for treatment and irradiate the neutron beam onto the treatment table 12 to treat an irradiated body taking a boron ($^{10}$B)-containing drug. Before the treatment, the treatment planning module 30 is configured to generate a treatment plan according to information such as medical image data of an irradiated site of the irradiated body and a parameter of the neutron beam N generated by the neutron generation device 11 for the treatment. In the irradiation for treatment, the control module 40 is configured to acquire a corresponding treatment plan of a present irradiated body from the treatment planning module 30, and control the neutron beam irradiation device 10 according to the treatment plan for the irradiation. In the embodiment, when the treatment planning module 30 is configured to generate the treatment plan, the parameter of the neutron beam N for the treatment is based on a neutron beam generated by a parameter of a radiation source.

Referring to FIG. 4, the neutron irradiation device 100 includes a radiation generation device 110 and a beam shaper 120. The radiation generation device 110 includes an accelerator 111, a beam transmission device 112, and a target T. The accelerator 111 is configured to accelerate charged particles (such as protons and deuterons) to generate a charged particle line P such as a proton line. The charged particle line P is irradiated onto the target T and generates a neutron line (a neutron beam) under an action of the target T. The target T is preferably a metal target. In the embodiment of the present disclosure, the target is made of lithium. However, as is known to those skilled in the art, the target may also be made of a metal material other than the lithium and beryllium, such as tantalum (Ta) or tungsten (W). The target may be a circular plate, and may also be other solid shapes, and may also be made of a liquid (liquid metal).

A collimator 126 for gathering the neutron beam is provided at an end of the beam shaper 120 close to the irradiated body M. The beam shaper 120 can adjust quality of the neutron beam. The neutron beam N generated by the radiation generation device 20 is irradiated onto the irradiated body M from the beam outlet 127 through the neutron beam of the beam shaper 120. The collimator 126 is configured to gather the neutron beam, such that the neutron beam has a high targeting ability in the treatment. It may be understood that in the embodiment, the collimator 126 may also not be provided, and the beam from the beam shaper 120 is directly irradiated onto the irradiated body M.

The beam shaper 120 further includes a reflector 121, a moderator 122, a thermal neutron absorber 123, a radiation shield 124, and a beam channel 125. The energy spectrum of neutrons generated by the radiation generation device 110 is very wide. Except that epithermal neutrons meet the treatment requirement, neutrons and photons of other types are to be reduced as much as possible, so as not to hurt the operator or the irradiated body. Hence, concerning neutrons from the target T, energies (>10 keV) of fast neutrons are adjusted by the moderator 2212 to an energy range (0.5 eV to 10 keV) of the epithermal neutrons, and the thermal neutrons (<0.5 eV) are reduced as much as possible. The moderator 2212 is made of a material having a large action section with the fast neutrons but a small action section with the epithermal neutrons, and preferably made of at least one of $D_2O$, $AlF_3$, Fluental™, $CaF_2$, $Li_2CO_3$, $MgF_2$, and $Al_2O_3$. The reflector 121 surrounds the moderator 122, and reflects neutrons diffused through the moderator 122 back to the neutron beam N, thereby improving the utilization rate of the neutrons. The reflector is made of a material with a strong neutron reflectivity, and preferably made of at least one of Pb or Ni. The thermal neutron absorber 123 is provided behind the moderator 122, and made of a material having a large action section with the thermal neutrons, preferably Li-6. The thermal neutron absorber is configured to absorb thermal neutrons passing through the moderator 122 to reduce the thermal neutrons in the neutron beam N, thereby preventing an excessive dose to the superficial normal tissue in the treatment. It may be understood that the thermal neutron absorber 123 may also be integrated with the moderator. The material of the moderator contains Li-6. The radiation shield 124 is configured to shield neutrons and photons leaked from a portion other than the beam channel 125. A material of the radiation shield 124 includes at least one of a photon shielding material and a neutron shielding material. As a preferred embodiment, the material of the radiation shield 124 includes lead (Pb) as the photon shielding material and polyethylene (PE) as the neutron shielding material. The beam outlet 127 is provided behind the beam channel 125. An epithermal neutron beam from the beam outlet 127 is irradiated onto the irradiated body. After passing through the superficial normal tissue, the epithermal neutron beam is moderated as thermal neutrons to reach tumor cells of the irradiated body M. In the irradiation for treatment, the control module 20 is configured to acquire a corresponding treatment plan of a present patient from the treatment planning module 10, and control the neutron irradiation device 100 according to the treatment plan for irradiation.

In the present disclosure, before the treatment, the radiolabeled boron-containing ($^{10}$B) drug is taken by the irradiated body, and boron ($^{10}$B) concentration associated information is acquired through a radionuclide medical image (such as a PET image). That is, medical image data on the irradiated site of the irradiated body includes tissue associated information and boron concentration associated information. Specifically, according to a radionuclide medical image scanning device, positrons generated by decay of the radionuclide take an electron -positron annihilation reaction with electrons in the tissue. By this time, gamma rays from the annihilation reaction can be detected by a detector such as a photomultiplier tube (PMT). Thereafter, a cross-sectional image reflecting a radioisotope distribution of the positrons is formed through a computer, thereby obtaining the boron concentration associated information. In an embodiment, $^{18}$F-BPA-PET is used as a marker for scanning. Through a counting rate for generating photons under an action of annihilation between the positron and the electron released by decay of $^{18}$F, original data generated by each lattice of the image is converted into an intensity of each lattice in the PET image to serve as an output of the medical image data. The $^{18}$F is labeled on the BPA. The original data on the PET image can be taken as a basis for quantifying $^{10}$B. It may be understood that if a boron-free drug that is radiolabeled and has a similar affinity to the tumor cells as the boron-containing drug is used for the radionuclide medical image scanning, the original data on the image may also be taken as a basis for quantifying $^{10}$B.

The SUV is a semi-quantitative index for acquiring the radionuclide medical image, and refers to a ratio of the radioactive activity of the developer (radiolabeled drug) taken by the local tissue to the average injected radioactive activity of the whole body. The SUV is specifically defined by:

$$SUV_{\text{body weight}}(kg/ml) = \frac{\text{Acitivity Concentration in ROI(Bq/ml)}}{\left(\frac{Injected\ Dose(Bq)}{body\ weight(kg)}\right)} \tag{Eq.1}$$

where, ROI is defined in the image, Acitivity Concentration in ROI is an average radioactive activity of each unit volume in the ROI, *Injected Dose* is an injected radioactive activity, and *body weight* is a body weight of the irradiated body.

In a first embodiment, the radionuclide medical image is a PET image, and the radiolabeled boron-containing drug taken by the irradiated body is a $^{18}$F-BPA. It may be understood that the $^{18}$F-BPA may further be replaced by other radiolabeled drugs or other boron-containing drugs, and may also be a boron-free drug that is radiolabeled and has a similar affinity to the tumor cells as the boron-containing drug, such as $^{18}$F-FDG.

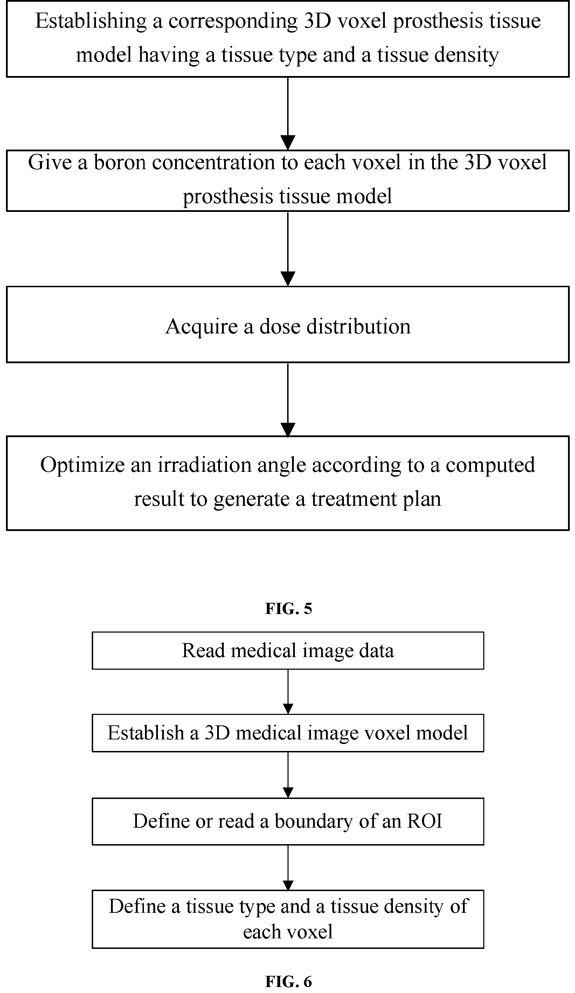

Referring to FIG. 5, the present disclosure provides a treatment plan generation method based on a BNCT system, including the following steps:

A 3D voxel prosthesis tissue model having a tissue type and a tissue density is established:

According to tissue associated information in medical image data of an irradiated site, the corresponding 3D voxel prosthesis tissue model having the tissue type and the tissue density is established, which is as shown in FIG. 6, and specifically includes the following steps:

The medical image data is read: The medical image data usually uses a digital imaging and communications in medicine (DICOM) format. The DICOM data includes information such as a body weight of the irradiated body, an injection dose, measure time for a drug activity, scan time, half time of radionuclide, and radiopharmaceutical. The information can be determined at the beginning of radionuclide medical image scanning. The information can be manually input by an operator, and may also be automatically acquired or invoked.

A 3D medical image voxel model is established.

A boundary of an ROI is determined.

A tissue type (element composition) and a tissue density of each voxel are defined.

The tissue type and the tissue density of the voxel may be defined automatically according to a conversion relationship between CT image data and the tissue type as well as the tissue density, and may also be defined manually by a user. For example, a specific tissue type and a specific tissue density are provided for each voxel within the boundary of the ROI.

Boron concentration assignment is performed on each voxel in the 3D voxel prosthesis tissue model.

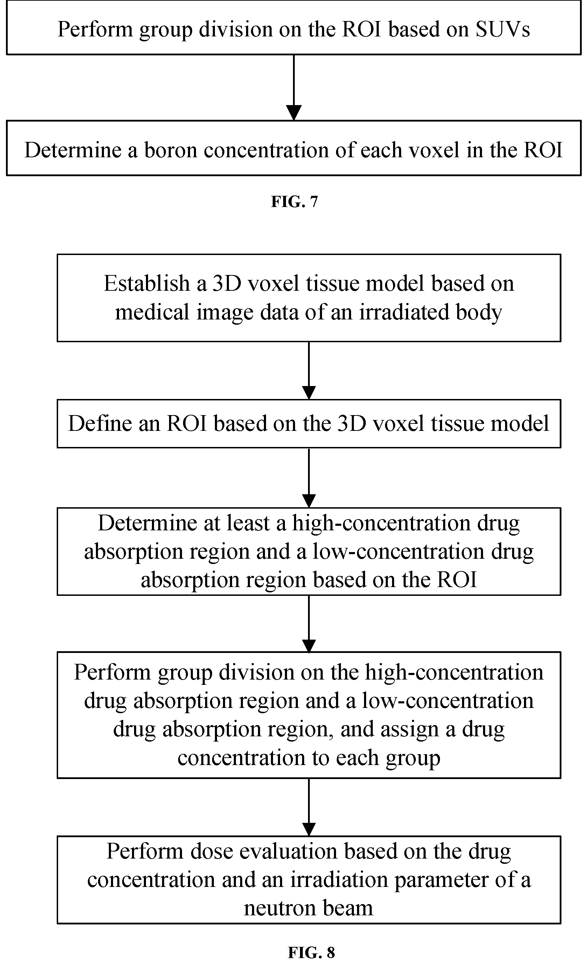

A corresponding boron concentration is given to each voxel in the 3D voxel prosthesis tissue model according to boron concentration associated information in the medical image data of the irradiated site. According to the 3D voxel prosthesis tissue model labeled with the tissue boron concentration information, a concentration of the boron-containing drug in each voxel can be known clearly. In simulation of neutron irradiation, collision trajectories and energy distributions of nuclear particles in the ROI of the irradiated body can be reflected more truly. The boron concentration associated information is acquired from a radionuclide medical image of the irradiated site. The radiolabeled boron-containing drug taken by the irradiated body is used for the radionuclide medical image scanning. Different boron concentrations are assigned by a treatment planning module 30 to each voxel in the 3D voxel prosthesis tissue model based on the radionuclide medical image data and the boron concentration information, which is as shown in FIG. 7, and specifically includes the following steps:

Group division is performed on the ROI based on SUVs.

Through the above Eq. 1, $^{10}$B information of each voxel in the ROI defined in the PET image can be converted into an SUV for quantitative analysis, thereby obtaining a maximum, a minimum, and an average of SUVs in the ROI. The SUVs are divided into I groups. An average of SUVs in an ith group after the group division is computed by:

$$\overline{SUV}_{ROI,i} = \frac{SUV_{ROI,upper} - SUV_{ROI,lower}}{I} \times (i - 0.5) + SUV_{ROI,lower} \qquad (Eq. 2)$$

$$i = 1, 2, 3, 4, ..., I$$

where, I is a number of groups, and is an even number in an embodiment of the present disclosure, $SUV_{ROI,upper}$ is an upper limit of SUVs in the ROI, and $SUV_{ROI,lower}$ is a lower limit of the SUVs in the ROI. The upper limit and the lower limit of the SUVs in the ROI are set based on a range of the SUVs in the ROI, and are adjusted based on grouping and normalization.

When the ROI is composed of M materials, a total number of the materials used is $M_{total} = M*I$ after the group division. If the I is too large, the operation process is time-consuming. If the I is too small, an actual distribution of boron in the ROI cannot be reflected truly. The drug and the body parameter of the irradiated body are obtained according to a tag (DICOM Tag) of the PET image. For the ROI defined by the user, an uptake degree of the drug and distribution information thereof are obtained, thereby determining the number of groups in the group division. If the uptake degree of the drug is distributed discretely, more groups are provided. On the contrary, when the uptake degree of the drug is distributed uniformly, less groups are provided. Usually, $I \in [10,500]$, preferably, $I \in [10,100]$, and more preferably, the I is 40, 44, 50, 54 or 60.

In principle, before and after the group division on the SUVs in the ROI, a total number of $^{10}$B atoms in the ROI is the same. Upon this, the number of groups or the average of SUVs is adjusted by Eq. 3 to ensure that the total number of $^{10}$B atoms after the group division is the same as the total number of $^{10}$B atoms before the group division:

$$\int_V SUV_{ROI}(V)dV = k \sum_i N_i \overline{SUV}_{ROI,i}$$

$$(Eq. 3)$$

$$k = \frac{\int_V SUV_{ROI}(V)dV}{\sum_i N_i \overline{SUV}_{ROI,i}}$$

where, $SUV_{ROI}(V)$ is an SUV of a Vth voxel in the ROI, $\int_V SUV_{ROI}(V)dV$ is a total value of SUVs before the group division, $N_i$ is a count of an ith group, k is a normalization factor, and $\sum_i N_i \overline{SUV}_{ROI,i}$ is a total value of SUVs after the group division.

The number I of groups or the average of SUVs is adjusted through the normalization factor k, until a difference between the total number of $^{10}$B atoms after the group division and the total number of $^{10}$B atoms before the group division is within a preset range. In an embodiment, the preset range is 5%.

It may be understood that when the k is used to adjust the number I of groups, the average of SUVs changes correspondingly, and when the k is used to adjust the average of SUVs, the number I of groups changes correspondingly.

A boron concentration of the voxel in the ROI is determined:

A number $N_{B10}$,group(V) of $^{10}$B atoms of the voxel in the ROI is computed by:

$$N_{B10,group}(V) = \xi \times k \times \overline{SUV_{ROI,i}}$$

$$i = \left\lceil \frac{I \times (SUV_{ROI}(V) - SUV_{ROI,lower})}{SUV_{ROI,upper} - SUV_{ROI,lower}} \right\rceil \qquad \text{(Eq. 4)}$$

where, $\xi$ is a constant conversion factor, and is configured to convert each of SUVs into a corresponding number of $^{10}$B atoms, i represents a group index, $SUV_{ROI,upper}$ is the upper limit of SUVs in the ROI, $SUV_{ROI,lower}$ is the lower limit of the SUVs in the ROI, and $SUV_{ROI}(V)$ is the SUV of the Vth voxel in the ROI.

A dose distribution is acquired:

After information such as the boron concentration (namely the number of boron atoms) and the boron density is defined to each voxel of the ROI in the 3D voxel prosthesis tissue model, a Monte Carlo simulation program (such as a Monte Carlo N particle transport code (MCNP)) is used for simulation. By sampling at different irradiation angles, a distribution of physical dose rates of the 3D voxel prosthesis tissue model at the different irradiation angles is simulated. That is, at different sampling irradiation angles, a physical dose received by each voxel in the 3D voxel prosthesis tissue model in unit time under irradiation of the defined beam is simulated, thereby obtaining the dose distribution. Assuming that $^4$He and $^7$Li generated by the capture reaction are deposited on the spot, the physical dose $D_{B10}$ of the boron in each voxel is computed by:

$$D_{B10}(V) = \frac{N_{B10}(V) \times RR_{B10} \times E_{cap}}{Mass(V)} \qquad \text{(Eq. 5)}$$

$$N_{B10}(V) = \xi \times SUV_{ROI}(V)$$

where, $N_{B10}(V)$ represents a number of $^{10}$B atoms in the vth voxel, $FR_{B10}$ is a reaction rate of each of the $^{10}$B atoms, Ecap is an energy released by the $^{10}$B atom in the capture reaction, Mass(V) is a mass (unit: kg) of the vth voxel, $\xi$ is the constant conversion factor, $SUV_{ROI}(V)$ is the SUV of the Vth voxel in the ROI, and the physical dose $D_{B10}$ of the boron has a unit of Gy.

Irradiation positions and irradiation angles of the beam are to be determined in the sampling. The irradiation positions and the irradiation angles may be determined in a forward algorithm or an inverse algorithm. In the forward algorithm, the irradiation positions depend on an in-vitro position, and may be sequentially sampled according to a fixed angle or distance for computation, and may also be obtained through random sampling. The angle of the beam may be set as a vector direction from the irradiation position to a centroid of the tumor or a deepest site of the tumor. In the inverse algorithm, the start position is determined within the range of the tumor, the start position may be the centroid of the tumor, the deepest site of the tumor or a site randomly selected within the range of the tumor. The angle of the beam may be determined through random sampling or sampling at a specified interval. The angle of the beam may also be screened in the sampling. For example, beam angle evaluation is made to select an optimal irradiation angle or remove an irradiation angle unimplemented for interference of the device.

An irradiation angle is optimized according to a computed result to generate a treatment plan:

With a maximum dose, an average dose or a prescribed dose of the ROI as a constraint, irradiation time corresponding to the irradiation angle in the sampling is sought. Based on the irradiation time, an equivalent dose distribution of the 3D voxel prosthesis tissue model at the irradiation angle in the sampling is obtained. With a DVH, an isodose curve, a dose table and the like, the equivalent dose distributions obtained by performing simulation and computation on the 3D voxel prosthesis tissue model at different irradiation angles and corresponding irradiation time are evaluated, thereby optimizing the implementable and desired treatment plan.

In the first embodiment, the treatment planning module 30 in the BNCT system is configured to perform group division on an ROI based on SUVs, assign a corresponding boron concentration to each voxel through computation, and perform dose simulation in combination with medical image data and a beam parameter to formulate a treatment plan, such that the distribution of boron atoms in the ROI better conforms to an actual condition. This can improve the accuracy in the model establishment and the dose computation, ensure the accuracy of the treatment plan, and guarantee the treatment effect. It may be understood that because of the expensive price of the boron-containing drug, the boron-containing drug is injected into the irradiated body once in actual treatment. Therefore, in other optional implementations, when the treatment plan is formulated, the boron concentration is preset, and the treatment plan is further generated based on the preset boron concentration.

A second embodiment of the present disclosure provides a BNCT system, including a neutron irradiation device 10, a treatment planning module 30, and a control module 40. The neutron irradiation device 10 is configured to generate a neutron beam and irradiate an irradiated body. The treatment planning module 30 is configured to determine at least a high-concentration drug absorption region and a low-concentration drug absorption region based on an ROI, and perform drug concentration assignment and dose evaluation on the high-concentration drug absorption region and the low-concentration drug absorption region to generate a treatment plan. The control module 40 is configured to control the neutron irradiation device 10 to execute the treatment plan. The treatment planning module 30 includes a model establishment module, a processing module, a dose evaluation module, and a treatment plan generation module. The model establishment module, the processing module, the dose evaluation module, and the treatment plan generation module are electrically connected in sequence for data transmission.

The model establishment module is configured to establish a 3D voxel tissue model based on medical image data. It may be understood that the BNCT system further includes an imaging device. The imaging device is configured to acquire the medical image data required by the treatment planning module 30.

The medical image data is fused image data of first image data and second image data. Specifically, the first image data is image data capable of displaying a tissue profile of the irradiated body. The second image data is image data capable of displaying a drug distribution in the irradiated body. The second image data is particularly radionuclide medical image data. The drug taken by or injected into the irradiated body contains a radioactive marker, so as to display the drug distribution in the irradiated body in the second image data. The second image data includes a marker parameter capable of describing a marker distribution.

In conventional neutron capture therapy, a single boron concentration is assigned to a lesion region, and high requirements are imposed on accuracy for dividing the ROI. ROIs divided from different medical images are different, which makes the dose computation inaccurate. If the single boron concentration is assigned, and the ROI is divided through single image data, the computed result is more inaccurate. For example, the lesion region is divided according to the first image data, and the target region having a high drug uptake is divided according to the second image data. When the lesion region is greater than the target region, a part of the lesion region does not absorb the corresponding drug obviously. When the lesion region is less than the target region, the normal region absorbs the drug excessively. In the above two cases, there is a deviation in the dose computation. Even though the lesion region is close to the target region, due to a difference between distribution uniformities of drug concentrations in the two regions and the like, a maximum dose and a minimum dose in the dose evaluation are deviated.

Therefore, in the second embodiment, the ROI is divided with the fused image. This prevents corresponding defects of different image data, can divide the ROI according to the clear tissue profile and the distribution of the absorbed drug, and divides the ROI more accurately. Meanwhile, the ROI is divided into the high-concentration drug absorption region and the low-concentration drug absorption region. This relieves influences of the accuracy for dividing the ROI on the dose computation, prevents the problem of inaccurate dose computation caused by the conventional single boron concentration assignment, and further makes the dose evaluation more accurate.

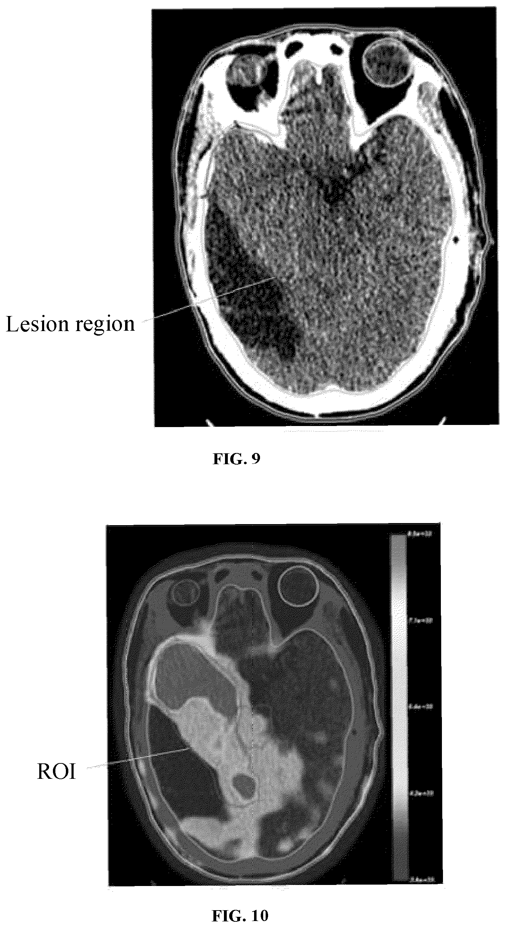

In some embodiments, the first image data is acquired by a first imaging device. The first imaging device is a CT device or a magnetic resonance (MR) device, and may specifically be a common CT device, an enhanced CT device, a CTsim device or the MR device. The CT device is more desirable to image a skeleton, while the MR device is more desirable to image a soft tissue. In the embodiment, the first imaging device may particularly use the CTsim device. Compared with other image acquisition devices, the image obtained by the CTsim device has a higher definition, thereby making image alignment and region division in subsequent steps more accurate. As shown in FIG. 9, FIG. 9 illustrates an initial lesion region divided according to the CTsim image. The first image data cannot display the drug distribution. When the first image data is acquired, the marker-containing drug may be taken by or injected into the irradiated body, and may also not be taken by or injected into the irradiated body.

In some embodiments, the second image data is acquired by a second imaging device. The second imaging device is particularly a radionuclide medical image acquisition device. The second imaging device is a multi-mode imaging device. The multi-mode imaging device includes at least a first mode unit and a second mode unit. The first mode unit can acquire a second functional image containing image data of the marker parameter. The second mode unit can acquire a second sectional image. One of the first mode unit and the second mode unit can acquire or generate a transformation matrix between the second functional image and the second sectional image. The transformation matrix can realize registration and fusion of the second functional image and the second sectional image. Further, with the transformation matrix, images acquired in same or different imaging methods are transformed, such that their spatial positions and spatial coordinates match with each other. That is, with respective imaging methods, two images are spatially registered, and the registered image data is fused to form a single image.

Further, the multi-mode imaging device may specifically be a combination of an emission computed tomography (ECT) device and other imaging devices, such as a PET-CT device combined with the PET device and the CT device, a single-photon emission computed tomography (SPECT)-CT device combined with the SPECT device and the CT device, or a PET-MR device combined with the PET device and the MR device.

In some embodiments, the second imaging device and the first imaging device may also be a same imaging device. The imaging device is a multi-mode imaging device, such as a PET-CT device, a SPECT-CT device or a PET-MR device. Data required by the first image data and the second image data can be acquired by those skilled in the art from image data output from the multi-mode imaging device.

The model establishment module is configured to perform 3D voxel tissue model establishment according to the first image data or the fused image data, and may be configured to establish the 3D voxel tissue model including the marker parameter. Each voxel in the model has a drug distribution parameter and a marker parameter correspondingly.

The processing module is configured to define an ROI based on the 3D voxel tissue model, determine at least a high-concentration drug absorption region and a low-concentration drug absorption region based on the ROI, and perform group division on the high-concentration drug absorption region and the low-concentration drug absorption region. The high-concentration drug absorption region is divided into at least one group. The low-concentration drug absorption region is divided into at least two groups. A drug concentration is assigned to each group.

The processing module can divide the ROI based on the fused image data. The ROI includes a region of a lesion to be irradiated for treatment. According to the marker parameter in the second image data, and in combination with the clear tissue profile of the irradiated body in the first image data, the processing module can divide the desired ROI accurately and automatically with the fused image data. As shown in FIG. 10, FIG. 10 illustrates an image fused by a CTsim image and a PET image. The divided ROI is delineated by a red line in FIG. 10. In some embodiments, the processing module 12 can be operated to divide the ROI.

The high-concentration drug absorption region and the low-concentration drug absorption region are divided based on a specified value that characterizes a drug concentration. The specified value is usually determined based on a therapeutic benefit.

In the high-concentration drug absorption region, the tissue has a higher uptake rate for the drug, and thus the drug has a high concentration. Drug concentrations of voxels in the high-concentration drug absorption region are all greater than the drug concentration characterized by the specified value. The drug concentrations in the high-concentration drug absorption region are distributed at a high uniformity. By assigning the uniform drug concentration in the region, the computed dose result better conforms to the actual acceptable dose. Hence, the high-concentration drug absorption region can be divided into at least one group.

In the low-concentration drug absorption region, the tissue has a lower uptake rate for the drug, and thus the drug has a low concentration. Drug concentrations of voxels in the low-concentration drug absorption region are all less than the drug concentration characterized by the specified value. The drug concentrations in the low-concentration drug absorption region are widely distributed at a poor uniformity. By directly assigning the uniform drug concentration in the region, the computed dose result is greatly varied from the actual acceptable dose. Hence, the low-concentration drug absorption region is divided into more regions to compute the dose based on the nonuniform drug concentrations, thereby making the dose evaluation more accurate and credible. The low-concentration drug absorption region can be divided into at least two groups. Further, a number of groups into which the high-concentration drug absorption region is divided is less than a number of groups into which the low-concentration drug absorption region is divided.

In some embodiments, the treatment planning module 30 is further configured to define a TBR as a ratio of a drug concentration in the ROI to a drug concentration in blood, and define a TNR as a ratio of the drug concentration in the ROI to a drug concentration in a normal tissue. The processing module is configured to divide the high-concentration drug absorption region and the low-concentration drug absorption region according to a value of the TBR or the TNR, and perform group division on the high-concentration drug absorption region and the low-concentration drug absorption region according to the value of the TBR or the TNR. The value of the TBR or the TNR serves as the above specified value.

Further, the processing module is configured to determine a region with a TBR greater than or equal to a first specified value or a region with a TNR greater than or equal to a second specified value in the ROI as the high-concentration drug absorption region. The processing module is configured to determine a region with a TBR less than the first specified value or a region with a TNR less than the second specified value in the ROI as the low-concentration drug absorption region.

In some embodiment, a drug taken by or injected into the irradiated body is a boron-containing drug. When a boron concentration in the normal tissue is lower than a boron concentration in the blood, specifically, a TNR is greater than a TBR in a same ROI, the first specified value is greater than or equal to 1.2, and the second specified value is greater than or equal to 1.5.

In some embodiments, the drug taken by or injected into the irradiated body is a BPA, and specifically a $^{18}$F-BPA. For the BPA, a boron concentration in the normal tissue is approximate to a boron concentration in the blood, specifically, a TNR is the same as a TBR in a same ROI, the first specified value is 2.5, and the second specified value is 2.5.

It may be understood that the TBR has an Nth specified value, the TNR has an Mth specified value, and the ROI is divided into N+1 or M+1 regions through the Nth specified value and the Mth specified value, N and M each being an integer greater than or equal to 1. A total number of divided regions, a number of regions into which the high-concentration drug absorption region is divided, and a number of regions into which the low-concentration drug absorption region is divided may be determined by factors such as the type of the drug, the distribution uniformity of the drug concentrations, the type of the tumor to be treated, and the individual difference of the irradiated body. The Nth specified value and the Mth specified value are specifically determined by factors such as the type of the specified value (whether it is the TBR or the TNR), the maximum specified value and the minimum specified value of the ROI, the type of the drug, the type of the tumor to be treated, and the individual difference of the irradiated body.

In the second embodiment, the processing module is configured to divide the ROI into three groups. The high-concentration drug absorption region is divided into one group, called a first region. The low-concentration drug absorption region is divided into two groups, respectively called a second region and a third region. The second region is a region with a TBR greater than or equal to a third specified value or a TNR greater than or equal to a fourth specified value in the low-concentration drug absorption region. The third region is a region with a TBR less than the third specified value or a TNR less than the fourth specified value in the low-concentration drug absorption region. The third specified value is greater than 1.5 and less than or equal to 2.0. The third specified value is the same as the fourth specified value. Further, when the drug is the BPA, the third specified value is the same as the fourth specified value, and is 2.0. It may be understood that the first specified value, the second specified value, the third specified value, and the fourth specified value each are a value of the TBR or the TNR.

By injecting a marker labeled drug into the irradiated body and acquiring the second image data, the drug distribution can be observed and obtained. Since the pharmacodynamics of the drug is not affected by the marker, the drug distribution obtained with the radionuclide marker is fairly credible.

In the embodiment of the present disclosure, the TBR or the TNR is acquired based on the radionuclide medical image such as the PET image, and the group division is performed on the ROI. Hence, the drug injected into the irradiated body is a radionuclide labeled drug. In other embodiments, the TBR or the TNR may also not be acquired through the medical image, and may be acquired by drawing blood or extracting a tissue to measure the drug concentration. In this case, the injected drug may be a common drug not labeled by the radionuclide.

The treatment planning module 30 is further configured to convert the drug concentration into one of a marker radioactive activity acquired based on the medical image data, a marker radioactive intensity, a number of atoms decayed in unit time, an effective count of annihilation photons, and an SUV for quantitative or semi-quantitative analysis, acquire a quantitative or semi-quantitative parameter of a corresponding marker, and label the quantitative or semi-quantitative parameter as the marker parameter. The specified value is the marker parameter. In some embodiments, the imaging device may directly acquire the marker parameter from the second image data or the fused image data. Preferably, the radionuclide marker may be $^{18}$F.

Based on the second image data, which is the PET image in the embodiment, an SUV of each region can be acquired. The TBR and the TNR may be respectively computed by Eq. (1) and Eq. (2):

$$TBR = \frac{SUV_i}{SUV_{blood}} \qquad (1)$$

$$TNR = \frac{SUV_i}{SUV_{normal}} \qquad (2)$$

where, $SUV_i$ is an SUV of an ith voxel in the ROI, $SUV_{blood}$ is an SUV in the blood, and $SUV_{normal}$ is an SUV in the normal tissue.

The processing module is configured to respectively assign drug concentrations to regions in each divided region. In some embodiments, a uniform drug concentration is assigned to each group of region. Specifically, based on a gross tumor volume (GTV) of a Vth region, the TBR or the TNR of each voxel in the region is averaged, and the computed result servers as an average specified value. Based on the average specified value, the uniform drug concentration of the region is obtained to serve as a basis for dose evaluation.

Further, the drug is a radionuclide labeled boron-containing drug. The treatment planning module 30 is configured to convert a boron concentration $N_{B10}$ into the SUV for the quantitative analysis:

$$N_{B10}(V) = \xi \times \overline{SUV_{ROI}(V)} \qquad \text{(Eq. 6)}$$

where, $N_{B10}(V)$ represents a number of $^{10}$B atoms in the Vth region, namely the boron concentration, $\xi$ is a constant conversion factor, and $SUV_{ROI}(V)$ is an average of SUVs in the Vth region of the ROI. The ROI is divided into V regions according to the above manner, V=1,2,3... Further, the processing module may be configured to automatically or manually define the boron concentration of each voxel in the 3D voxel tissue model according to a conversion relationship between the SUV and the boron concentration, and compute a physical dose of boron in the voxel based on the boron concentration.

In some embodiments, the dose evaluation module is configured to perform the dose evaluation based on the drug concentration and an irradiation parameter of the neutron beam. The treatment plan generation module is configured to generate the treatment plan based on a result of the dose evaluation.

The dose evaluation module is configured to simulate collision trajectories and energy distributions of nuclear particles in an internal 3D space through an internal preset Monte Carlo simulation program when the patient is irradiated by the neutron beam in the BNCT. That is, the dose evaluation module may be configured to simulate a distribution of physical dose rates, and compute a distribution of equivalent dose rates according to the distribution of physical dose rates. An irradiation parameter (such as a beam energy, a beam intensity, and a beam radius) of the neutron beam is defined in the Monte Carlo simulation program. A physical dose of each voxel of the 3D voxel tissue model in unit time under the defined irradiation parameter is simulated. In the dose computation of the BNCT, factors mainly contributing to the dose include a boron dose, a neutron dose, etc. In order to evaluate the dose in the BNCT more intuitively, different weighted factors are given to the dose composition for dose evaluation. For example, the weighted factors such as a relative biological effectiveness (RBE) and a compound biological effectiveness (CBE) are given to obtain a biologically equivalent dose, as shown by Eq. 7:

$$BNCT_{dose} = D_B \cdot CBE + D_n \cdot RBE_n + D_\gamma \cdot RBE_\gamma \qquad \text{(Eq. 7)}$$

where, $BNCT_{dose}$ is a total equivalent dose under the neutron irradiation, $D_B$ is a physical dose of the boron, $CBE$ is a compound biological effectiveness in a unit concentration of the boron-containing drug, $D_n$ is a physical dose of the neutron, $RBE_n$ is a relative biological effectiveness of the neutron, $D_\gamma$ is a physical dose of a photon, and $RBE_\gamma$ is a relative biological effectiveness of the photon. In some embodiments, CBE=3.8, RBE$_n$=3.2, and RBE$_\gamma$=1.0 can be substituted into the dose of the ROI for computation and evaluation.

In some embodiments, the equivalent dose distribution computed in the simulation of the 3D voxel tissue model can be evaluated with a mathematical algorithm such as a DVH, an isodose curve, and a dose table.

As shown in FIG. 8, FIG. 8 is a flowchart of the dose evaluation in the BNCT system according to the second embodiment of the present disclosure. The dose evaluation includes the following steps:

Model establishment: A 3D voxel tissue model is established based on medical image data of an irradiated body. Specifically, the step that the 3D voxel tissue model is established based on the medical image data further includes: First image data is acquired. The 3D voxel tissue model is established based on the first image data. Further, the first image data is image data capable of displaying a tissue profile of the irradiated body.

Division of an ROI: The ROI is defined based on the 3D voxel tissue model. Specifically, the step that the ROI is defined based on the 3D voxel tissue model further includes: The first image data is acquired. Second image data is acquired. Registration and alignment are performed on the first image data and the second image data to acquire fused image data. The ROI is divided based on the fused image data. The first image data is CT data or MRI data, and the second image data is radionuclide medical image data.

It may be understood that the step that the 3D voxel tissue model is established based on the first image data, the step that the second image data is acquired, and the step that the fused image data is acquired are executed in a random order. It may be understood that the 3D voxel tissue model may also be established based on the fused image data.

Region division and group division: At least a high-concentration drug absorption region and a low-concentration drug absorption region are determined based on the ROI. Group division is performed on the high-concentration drug absorption region and the low-concentration drug absorption region. The high-concentration drug absorption region is divided into at least one group, the low-concentration drug absorption region is divided into at least two groups. A drug concentration is assigned to each group.

Specifically, the region division and the group division further include the following steps:

Region division based on a TBR or a TNR: The TBR is defined as a ratio of a drug concentration in the ROI to a drug concentration in blood, and the TNR is defined as a ratio of the drug concentration in the ROI to a drug concentration in a normal tissue. A region with a TBR greater than or equal to a first specified value or a region with a TNR greater than or equal to a second specified value in the ROI is determined as the high-concentration drug absorption region. A region with a TBR less than the first specified value or a region with a TNR less than the second specified value in the ROI is determined as the low-concentration drug absorption region.

Determination of a specified value according to a drug: When a drug is a boron-containing drug, the first specified value is greater than or equal to 1.2, and the second specified value is greater than or equal to 1.5. When the drug is a BPA, the first specified value is the same as the second specified value, and is 2.5. Further, when the drug is the BPA, the region division based on the TBR or the TNR further includes: The ROI is divided into three groups. The high-concentration drug absorption region is divided into one group, called a first region. The low-concentration drug absorption region is divided into two groups, respectively called a second region and a third region. The second region is a region with a TBR greater than or equal to a third specified value or a TNR greater than or equal to a fourth specified value in the low-concentration drug absorption region. The third region is a region with a TBR less than the third specified value or a TNR less than the fourth specified value in the low-concentration drug absorption region. The third specified value is the same as the fourth specified value, and is 2.0.

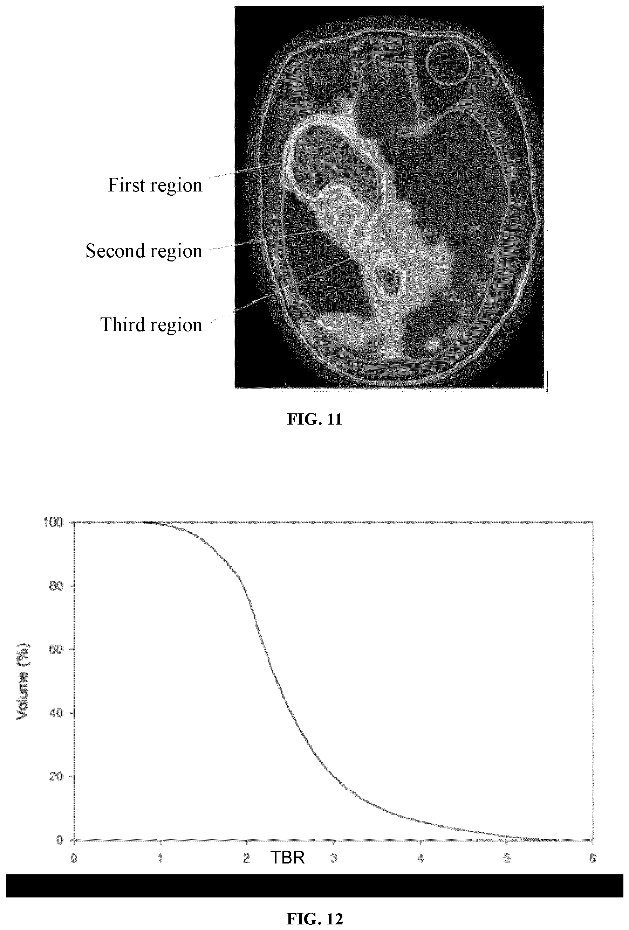

In some embodiments, the irradiated body is a patient with a brain tumor. Due to a large GTV $GTV_{anatomy}$ of the patient, the ROI has a volume of 155.9c.c., namely the $GTV_{anatomy}$ is 155.9c.c. The boron-containing drug $^{18}F$-BPA is injected into the patient, and the TBR acquired from the image data is in a range of 0.70-5.95. As shown in FIG. 11, the first specified value is 2.5. A region with a TBR≥2.5 is divided into the high-concentration drug absorption region and taken as the first region. The first region is a red region delineated by a green line in FIG. 11. A region with a TBR<2.5 is divided into the low-concentration drug absorption region. The third specified value is 2.0. The low-concentration drug absorption region is divided into the second region with 2.0≤TBR<2.5, and the third region with TBR<2.0. In FIG. 11, the second region is an orange region delineated by a yellow line, and the third region is a green region delineated by a red line.

Drug concentration assignment: Based on a $GTV_{anatomy}$ of a Vth region, TBRs or TNRs of voxels in the region are averaged to obtain a uniform boron concentration of the region.

In the embodiment of the patient, the first region has a $GTV_1$ of 60.8c.c. Based on a TBR-volume histogram shown in FIG. 12, an average specified value (average TBR) in the region is 3.30. The second region has a $GTV_2$ of 63.0c.c. An average TBR in the region based on FIG. 12 is 2.26. The third region has a $GTV_3$ of 35.1c.c. An average TBR in the region based on FIG. 12 is 1.67. A uniform boron concentration distribution is used for assignment. Each average TBR corresponds to one boron concentration.

Dose evaluation: The dose evaluation is performed based on the drug concentration and an irradiation parameter of the neutron beam.

Specifically, the dose evaluation includes: An irradiation dose is computed based on the drug concentration and the irradiation parameter of the neutron beam.

For the above patient, a dose computed with the existing treatment planning system NeuMANTA in a nonuniform boron concentration distribution is taken as a control group, and compared with a dose evaluation result provided by the second embodiment. In the computation of the control group, all boron concentrations in the $GTV_{anatomy}$ are grouped in 132 regions, and respectively assigned to the 132 regions. That is, the $GTV_{anatomy}$ is divided into the 132 regions to compute a dose in the uniform boron concentration distribution. In the second embodiment, the $GTV_{anatomy}$ is divided into three regions to compute the dose in the uniform boron concentration distribution.

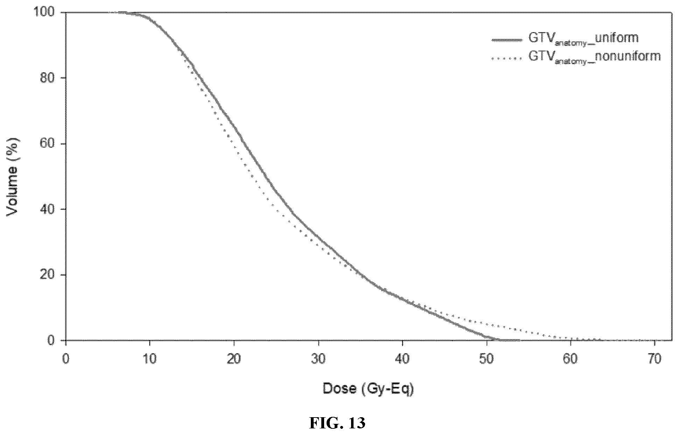

The dose computation time in the second embodiment is reduced by nearly 80% as compared to the dose computation time in the control group. In the computation of the control group, the boron concentrations in some of the 132 regions differ slightly, so the assignment of the different boron concentrations has a little impact on the dose computation, but increases the computation time, and takes up a large memory. For the region division and the group division in the second embodiment of the present disclosure, while the dose result is highly credible and highly accurate, the dose computation time is greatly reduced. This reduces time for formulating the treatment plan, lowers requirements and dependencies of the treatment planning system 10 on a hardware device, and makes the treatment more convenient. The dose for the $GTV_{anatomy}$ in the second embodiment of the present disclosure and the dose for the $GTV_{anatomy}$ in the control group and their difference are as shown in FIG. 13 and a table below:

|  | $D_{max}$(Gy-Eq) | $D_{min}$(Gy-Eq) | $D_{mean}$(Gy-Eq) | $D_{80}$(Gy-Eq) |
|---|---|---|---|---|
| In the embodiment of the present disclosure | 53.88 | 6.37 | 25.68 | 16.02 |
| Control group | 70.89 | 5.59 | 25.36 | 15.37 |
| Difference | -24.0% | 14.0% | 1.3% | 4.2% |

In the foregoing table, $D_{max}$ is a maximum dose acquired in simulated treatment of the irradiated body, $D_{min}$ is a minimum dose acquired in the third region of the irradiated body, $D_{mean}$ is an average dose acquired in the third region of the irradiated body, and $D_{80}$ is a dose acquired by 80% of a volume of a lesion, in a unit of Gy-Eq. In FIG. 13, the solid line represents the DVH of the method in the second embodiment of the present disclosure, the dotted line represents the DVH of the method in the control group, the horizontal axis Dose(Gy-Eq) is the irradiated dose, and the longitudinal axis Volume(%) is a volume of the lesion by percent.

As can be seen from the above table and FIG. 13, the DVHs in the two methods are fairly consistent. Specifically, the deviation between the result computed with the method in the second embodiment of the present disclosure and the result computed with the nonuniform boron concentration is not greater than 15% in $D_{min}$, $D_{mean}$ and $D_{80}$. Particularly, in $D_{mean}$ and $D_{80}$, the deviation between the result computed with the uniform boron concentration and the result computed with the nonuniform boron concentration is not greater than 5%. In order to better control the lesion in actual treatment, the dose in actual treatment usually takes the low dose as a reference basis, and is based on the dose $D_{80}$ acquired for 80% of the volume of the lesion for example. This ensures that the lesion can receive an enough prescribed dose, and reduces influences on the physical health of the patient. As can be seen, with the $D_{80}$ as an evaluation standard, there is a little deviation between the result computed in the embodiment of the present disclosure and the result computed with the nonuniform boron concentration in the control group. Therefore, the method in the present disclosure saves the computation time, and can ensure accuracy of the dose computation.

It may be understood that the dose evaluation method can be applied to the BNCT system.

It should be understood that although the steps in the flowcharts in the above embodiments are shown in sequence as indicated by the arrows, these steps are not necessarily performed in sequence as indicated by the arrows. The execution order of these steps is not strictly limited, and these steps may be executed in other orders, unless clearly described otherwise. Moreover, at least some of the steps in the flowcharts in the above embodiments may include a plurality of steps or stages. The steps or stages are unnecessarily executed at the same time, but may be executed at different times. The execution order of the steps or stages is unnecessarily carried out sequentially, but may be executed alternately with other steps or at least some of the steps or stages of other steps.

The technical characteristics of the above embodiments can be employed in arbitrary combinations. To provide a concise description of these embodiments, all possible combinations of all the technical characteristics of the above embodiments may not be described; however, these combinations of the technical characteristics should be construed as falling within the scope defined by the specification as long as no contradiction occurs. The above embodiments are merely illustrative of several implementations of the present disclosure, and the description thereof is more specific and detailed, but is not to be construed as a limitation to the patentable scope of the present disclosure. It should be noted that, for a person of ordinary skill in the art, several variations and improvements can be made without departing from the concept of the present disclosure, all of which fall within the protection scope of the present disclosure. Therefore, the protection scope of the present disclosure should be subject to the protection scope defined by the claims.

**Claims**

1. A boron neutron capture therapy (BNCT) system, comprising:

   a neutron beam irradiation device configured to generate a neutron beam and irradiate an irradiated body;
   a treatment planning module configured to perform boron concentration assignment on each voxel in a three-dimensional (3D) voxel prosthesis tissue model and generate a treatment plan; and
   a control module configured to control the neutron beam irradiation device according to the treatment plan for irradiation.

2. The BNCT system according to claim 1, wherein the treatment planning module is configured to perform group division on a region of interest (ROI) in the 3D voxel prosthesis tissue model based on standardized uptake values (SUVs) and compute an average of SUVs in each group.

3. The BNCT system according to claim 2, wherein the average of SUVs in each group is computed by:

$$\overline{SUV}_{ROI,i} = \frac{SUV_{ROI,upper} - SUV_{ROI,lower}}{I} \times (i - 0.5) + SUV_{ROI,lower} \qquad \text{(Eq. 2)}$$

$$i = 1, 2, 3, 4, ..., I$$

   wherein, I is a number of groups, and is an even number in an embodiment of the present disclosure, $SUV_{ROI,upper}$ is an upper limit of SUVs in the ROI, and $SUV_{ROI,lower}$ is a lower limit of the SUVs in the ROI.

4. The BNCT system according to claim 3, wherein the number I of groups is greater than or equal to 10 and less than or equal to 500.

5. The BNCT system according to claim 3, wherein the number I of groups is greater than or equal to 10 and less than or equal to 100.

6. The BNCT system according to claim 3, wherein the number I of groups is 40, 44, 50, 54 or 60.

7. The BNCT system according to claim 3, wherein the treatment planning module is configured to adjust the number I of groups or the average of SUVs based on a normalization factor k; and the normalization factor k is computed by:

$$\int_V SUV_{ROI}(V)dV = k \sum_i N_i \overline{SUV}_{ROI,i}$$

$$(\text{Eq. 3})$$

$$k = \frac{\int_V SUV_{ROI}(V)dV}{\sum_i N_i \overline{SUV}_{ROI,i}}$$

wherein, $SUV_{ROI}(V)$ is an SUV of a Vth voxel in the ROI, $\int_V SUV_{ROI}(V)dV$ is a total value of SUVs before the group division, $N_i$ is a count of an ith group, k is the normalization factor, and $\Sigma_i N_i \overline{SUV}_{ROI,i}$ is a total value of SUVs after the group division.

8. The BNCT system according to claim 7, wherein the treatment planning module is configured to determine a boron concentration $N_{B10}$,group(V) of each voxel in the ROI by:

$$N_{B10,group}(V) = \xi \times k \times \overline{SUV}_{ROI,i}$$

$$i = \left\lceil \frac{I \times (SUV_{ROI}(V) - SUV_{ROI,lower})}{SUV_{ROI,upper} - SUV_{ROI,lower}} \right\rceil$$

$$(\text{Eq. 4})$$

wherein, $\xi$ is a constant conversion factor, and is configured to convert each of SUVs into a corresponding number of $^{10}B$ atoms, i represents a group index, and $SUV_{ROI}(V)$ is the SUV of the Vth voxel in the ROI.

9. The BNCT system according to claim 1, wherein the treatment planning module is configured to determine at least a high-concentration drug absorption region and a low-concentration drug absorption region based on an ROI of the irradiated body, and perform drug concentration assignment and dose evaluation on the high-concentration drug absorption region and the low-concentration drug absorption region to generate the treatment plan.

10. The BNCT system according to claim 9, wherein the treatment planning module comprises:

a model establishment module configured to establish a 3D voxel tissue model based on medical image data of the irradiated body;
a processing module configured to define the ROI based on the 3D voxel tissue model, determine at least the high-concentration drug absorption region and the low-concentration drug absorption region based on the ROI, perform group division on the high-concentration drug absorption region and the low-concentration drug absorption region, the high-concentration drug absorption region being divided into at least one group, the low-concentration drug absorption region being divided into at least two groups, and assign a drug concentration to each group;
a dose evaluation module configured to perform the dose evaluation based on the drug concentration and an irradiation parameter of the neutron beam; and
a treatment plan generation module configured to generate the treatment plan based on a result of the dose evaluation.

11. The BNCT system according to claim 10, wherein the treatment planning module is further configured to define a tumor-to-blood ratio (TBR) as a ratio of a drug concentration in the ROI to a drug concentration in blood, and define a tumor-to-normal tissue ratio (TNR) as a ratio of the drug concentration in the ROI to a drug concentration in a normal tissue; and
the processing module is configured to determine a region with a TBR greater than or equal to a first specified value or a region with a TNR greater than or equal to a second specified value in the ROI as the high-concentration drug absorption region, and determine a region with a TBR less than the first specified value or a region with a TNR less than the second specified value in the ROI as the low-concentration drug absorption region.

12. The BNCT system according to claim 11, wherein a drug is a boron-containing drug, the first specified value is greater than or equal to 1.2, and the second specified value is greater than or equal to 1.5.

13. The BNCT system according to claim 12, wherein the drug is a boronophenylalanine (BPA), the first specified value is 2.5, and the second specified value is 2.5.

14. The BNCT system according to claim 13, wherein the processing module is configured to divide the ROI into three groups; the high-concentration drug absorption region is divided into one group, called a first region; the low-concentration drug absorption region is divided into two groups, respectively called a second region and a third region; the second region is a region with a TBR greater than or equal to a third specified value or a TNR greater than or equal to a fourth specified value in the low-concentration drug absorption region; the third region is a region with a TBR less than the third specified value or a TNR less than the fourth specified value in the low-concentration drug absorption region; the third specified value is greater than 1.5 and less than or equal to 2.0; and the fourth specified value is the same as the third specified value.

15. The BNCT system according to claim 14, wherein the fourth specified value is the same as the third specified value, and is 2.0.

16. The BNCT system according to claim 12, wherein the drug is a radionuclide labeled boron-containing drug; and the treatment planning module is further configured to convert the drug concentration into one of a marker radioactive activity acquired based on the medical image data, a marker radioactive intensity, a number of atoms decayed in unit time, an effective count of annihilation photons, and an SUV for quantitative or semi-quantitative analysis.

17. The BNCT system according to claim 16, wherein the treatment planning module is configured to convert the drug concentration into the SUV for the quantitative analysis:

$$N_{B10}(V) = \xi \times \overline{SUV_{ROI}(V)} \qquad \text{(Eq. 6)}$$

wherein, $N_{B10}(V)$ represents a number of $^{10}B$ atoms in a Vth region, $\xi$ is a constant conversion factor, and $\overline{SUV_{ROI}(V)}$ is an average of SUVs in the Vth region of the ROI.

18. A treatment plan generation method of a boron neutron capture therapy (BNCT) system, comprising:

establishing a three-dimensional (3D) voxel prosthesis tissue model having a tissue type and a tissue density;
performing boron concentration assignment on each voxel in the 3D voxel prosthesis tissue model;
performing dose evaluation based on the boron concentration assignment and an irradiation parameter of a neutron beam to obtain a dose distribution; and
optimizing an irradiation angle according to a computed result to generate a treatment plan.

19. The treatment plan generation method according to claim 18, wherein the establishing a 3D voxel prosthesis tissue model having a tissue type and a tissue density comprises:

reading medical image data;
establishing a 3D medical image voxel model;
defining a boundary of a region of interest (ROI); and
defining a tissue type, namely, element composition, and a tissue density of each voxel.

20. The treatment plan generation method according to claim 19, wherein the performing boron concentration assignment on each voxel in the 3D voxel prosthesis tissue model comprises:

performing group division on the ROI based on standard uptake values (SUVs); and
determining a boron concentration of the voxel in the ROI.

21. The treatment plan generation method according to claim 20, wherein the performing group division on the ROI comprises: converting $^{10}B$ information of each voxel in the ROI of an image into one of the SUVs by:

$$SUV_{\text{body weight}}(\text{kg/ml}) = \frac{\text{Acitivity Concentration in ROI(Bq/ml)}}{\left(\frac{Injected\ Dose(Bq)}{body\ weight(kg)}\right)} \qquad (\text{Eq.1})$$

wherein, ROI is defined in the image, Acitivity Concentration in ROI is an average radioactive activity of each unit volume in the ROI, *Injected Dose* is an injected radioactive activity, and *body weight* is a body weight of the irradiated body.

22. The treatment plan generation method according to claim 18, wherein the dose evaluation comprises:

establishing a 3D voxel tissue model based on medical image data of an irradiated body;
defining an ROI based on the 3D voxel tissue model;
determining at least a high-concentration drug absorption region and a low-concentration drug absorption region based on the ROI, performing group division on the high-concentration drug absorption region and the low-concentration drug absorption region, the high-concentration drug absorption region being divided into at least one group, and the low-concentration drug absorption region being divided into at least two groups, and assigning a drug concentration to each group; and
performing dose evaluation based on the drug concentration and the irradiation parameter of the neutron beam.

23. The treatment plan generation method according to claim 22, wherein the determining at least a high-concentration drug absorption region and a low-concentration drug absorption region based on the ROI comprises: defining a tumor-to-blood ratio (TBR) as a ratio of a drug concentration in the ROI to a drug concentration in blood, and defining a tumor-to-normal tissue ratio (TNR) as a ratio of the drug concentration in the ROI to a drug concentration in a normal tissue; and determining a region with a TBR greater than or equal to a first specified value or a region with a TNR greater than or equal to a second specified value in the ROI as the high-concentration drug absorption region, and determining a region with a TBR less than the first specified value or a region with a TNR less than the second specified value in the ROI as the low-concentration drug absorption region.

24. The treatment plan generation method according to claim 23, wherein a drug is a boron-containing drug, the first specified value is greater than or equal to 1.2, and the second specified value is greater than or equal to 1.5.

25. The treatment plan generation method according to claim 24, wherein the drug is a boronophenylalanine (BPA), and the first specified value is the same as the second specified value, and is 2.5.

26. The treatment plan generation method according to claim 25, wherein the ROI is divided into three groups; the high-concentration drug absorption region is divided into one group, called a first region; the low-concentration drug absorption region is divided into two groups, respectively called a second region and a third region; the second region is a region with a TBR greater than or equal to a third specified value or a TNR greater than or equal to a fourth specified value in the low-concentration drug absorption region; the third region is a region with a TBR less than the third specified value or a TNR less than the fourth specified value in the low-concentration drug absorption region; and the third specified value is the same as the fourth specified value, and the third specified value is greater than 1.5 and less than or equal to 2.0.

27. The treatment plan generation method according to claim 26, wherein the drug is a radionuclide labeled boron-containing drug; and the drug concentration is converted into the SUV for quantitative analysis:

$$N_{B10}(V) = \xi \times \overline{SUV_{ROI}(V)} \qquad (\text{Eq. 6})$$

wherein, $N_{B10}(V)$ represents a number of $^{10}B$ atoms in a Vth region, $\xi$ is a constant conversion factor, and $\overline{SUV_{ROI}(V)}$ is an average of SUVs in the Vth region of the ROI.

**FIG. 1**

$$^{1}n + {}^{10}B \longrightarrow {}^{11}B* \begin{cases} {}^{7}Li + {}^{4}He + 2.79\ MeV & (6.1\ \%) \\ {}^{7}Li* + {}^{4}He + 2.31\ MeV & (93.9\ \%) \\ \quad \downarrow \\ {}^{7}Li + \gamma\text{-ray } (0.48\ MeV) \end{cases}$$

**FIG. 2**

Neutron beam irradiation device 10

| Neutron generation device 11 | → | Treatment table 12 | | Treatment planning module 30 | | Control module 40 |

**FIG. 3**

**FIG. 4**

Establishing a corresponding 3D voxel prosthesis tissue model having a tissue type and a tissue density

Give a boron concentration to each voxel in the 3D voxel prosthesis tissue model

Acquire a dose distribution

Optimize an irradiation angle according to a computed result to generate a treatment plan

**FIG. 5**

Read medical image data

Establish a 3D medical image voxel model

Define or read a boundary of an ROI

Define a tissue type and a tissue density of each voxel

**FIG. 6**

Perform group division on the ROI based on SUVs

Determine a boron concentration of each voxel in the ROI

**FIG. 7**

Establish a 3D voxel tissue model based on medical image data of an irradiated body

Define an ROI based on the 3D voxel tissue model

Determine at least a high-concentration drug absorption region and a low-concentration drug absorption region based on the ROI

Perform group division on the high-concentration drug absorption region and a low-concentration drug absorption region, and assign a drug concentration to each group

Perform dose evaluation based on the drug concentration and an irradiation parameter of a neutron beam

**FIG. 8**

Lesion region

**FIG. 9**

ROI

**FIG. 10**

First region

Second region

Third region

**FIG. 11**

**FIG. 12**

**FIG. 13**

## INTERNATIONAL SEARCH REPORT

| International application No. |
| --- |
| **PCT/CN2023/129862** |

| A. | CLASSIFICATION OF SUBJECT MATTER |
| --- | --- |

A61N5/10(2006.01)i

According to International Patent Classification (IPC) or to both national classification and IPC

| B. | FIELDS SEARCHED |
| --- | --- |

Minimum documentation searched (classification system followed by classification symbols)

IPC：A61N5/-

Documentation searched other than minimum documentation to the extent that such documents are included in the fields searched

Electronic data base consulted during the international search (name of data base and, where practicable, search terms used)

DWPI, WPABS, CNTXT, ENTXT, CNKI: 中硼, 硼, 中子, 治疗, 计划, 三维, 体素, 模型, 赋值, 高, 低, 浓度, 分组, 计算, 剂量, 分布, 角度, Boron, neutron, treatment, plan, three-dimensional, voxel, model, dose, angle, concentration, distribution

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 114367061 A (NEUBORON THERAPY SYSTEM LTD.) 19 April 2022 (2022-04-19) description, paragraphs [0043]-[0095], and figures 1-7 | 1-8, 18-21 |
| Y | CN 114367061 A (NEUBORON THERAPY SYSTEM LTD.) 19 April 2022 (2022-04-19) description, paragraphs [0043]-[0095], and figures 1-7 | 9-17, 22-27 |
| Y | CN 110013613 A (DONGGUAN DONGYANGGUANG GAONENG MEDICAL EQUIPMENT CO., LTD.) 16 July 2019 (2019-07-16) description, paragraphs [0006]-[0011], and figure 1 | 9-17, 22-27 |
| X | CN 113877073 A (NEUBORON THERAPY SYSTEM LTD.) 04 January 2022 (2022-01-04) description, paragraphs [0034]-[0074], and figures 1-7 | 1-8, 18-21 |
| A | CN 104548372 A (SHANGHAI UNITED IMAGING HEALTHCARE CO., LTD.) 29 April 2015 (2015-04-29) entire document | 1-27 |
| A | CN 109308733 A (NEUBORON MEDTECH LTD.) 05 February 2019 (2019-02-05) entire document | 1-27 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **12 December 2023** | **20 December 2023** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/129862**

**C.    DOCUMENTS CONSIDERED TO BE RELEVANT**

| Category* | Citation of document, with indication, where appropriate, of the relevant passages | Relevant to claim No. |
|---|---|---|
| A | CN 110327554 A (SOUTHERN MEDICAL UNIVERSITY) 15 October 2019 (2019-10-15)<br>entire document | 1-27 |
| A | CN 115131376 A (LANZHOU UNIVERSITY) 30 September 2022 (2022-09-30)<br>entire document | 1-27 |
| A | CN 111494810 A (RAYSEARCH LABORATORIES AB) 07 August 2020 (2020-08-07)<br>entire document | 1-27 |
| A | EP 1658878 A1 (THE EUROPEAN COMMUNITY, REPRESENTED BY THE EUROPEAN COMMISSION) 24 May 2006 (2006-05-24)<br>entire document | 1-27 |

Form PCT/ISA/210 (second sheet) (July 2022)

**INTERNATIONAL SEARCH REPORT**
Information on patent family members

International application No.

**PCT/CN2023/129862**

| Patent document cited in search report | | | Publication date (day/month/year) | Patent family member(s) | | | Publication date (day/month/year) |
|---|---|---|---|---|---|---|---|
| CN | 114367061 | A | 19 April 2022 | WO | 2022078175 | A1 | 21 April 2022 |
| | | | | US | 2023241414 | A1 | 03 August 2023 |
| | | | | CN | 114367061 | B | 23 June 2023 |
| CN | 110013613 | A | 16 July 2019 | | None | | |
| CN | 113877073 | A | 04 January 2022 | WO | 2022001594 | A1 | 06 January 2022 |
| | | | | EP | 4166193A1 | A1 | 19 April 2023 |
| | | | | JP | 2023531798 | A | 25 July 2023 |
| CN | 104548372 | A | 29 April 2015 | CN | 104548372 | B | 22 December 2017 |
| CN | 109308733 | A | 05 February 2019 | | None | | |
| CN | 110327554 | A | 15 October 2019 | CN | 110327554 | B | 10 November 2020 |
| CN | 115131376 | A | 30 September 2022 | | None | | |
| CN | 111494810 | A | 07 August 2020 | EP | 3677309 | A1 | 08 July 2020 |
| | | | | EP | 3677309 | B1 | 10 March 2021 |
| | | | | JP | 2020108754 | A | 16 July 2020 |
| | | | | JP | 6803962 | B2 | 23 December 2020 |
| | | | | US | 2020215352 | A1 | 09 July 2020 |
| | | | | US | 10850125 | B2 | 01 December 2020 |
| | | | | CN | 111494810 | B | 29 October 2021 |
| EP | 1658878 | A1 | 24 May 2006 | | None | | |

Form PCT/ISA/210 (patent family annex) (July 2022)